# NOUVEAU FASCICULE DE BREVET EUROPEEN

(11) **EP 1 330 266 B2**
(45) Date de publication et mention de la décision concernant l'opposition: **26.08.2009**
(45) Mention de la délivrance du brevet: 11.05.2005
(21) Numéro de dépôt: 01980600.9
(22) Date de dépôt: 19.10.2001
(51) Int. Cl.: A61K 47/48

(54) **PROCEDE DE FABRICATION DE TRES FINES PARTICULES CONSTITUEES D'UN PRINCIPE INSERE DANS UNE MOLECULE HOTE**
VERFAHREN ZUR HERSTELLUNG VON SEHR FEINEN TEILCHEN BESTEHEND AUS EINEM WIRTSMOLEKÜL MIT INSERIERTEM WIRKSTOFF
METHOD FOR MAKING VERY FINE PARTICLES CONSISTING OF A PRINCIPLE INSERTED IN A HOST MOLECULE

(30) Priorité: 19.10.2000 FR 0013393
(43) Date de publication de la demande: 30.07.2003
(73) Titulaire: SEPAREX, F-54250 Champigneulles (FR)
(72) Inventeur: PERRUT, Michel, F-54000 Nancy (FR); JUNG, Jennifer, F-54000 Nancy (FR); LEBOEUF, Fabrice, F-54000 Nancy (FR); FABING, Isabelle, F-54000 Nancy (FR)
(74) Mandataire: Puiroux, Guy
(86) Numéro de dépôt international: PCT/FR2001/003238
(87) Numéro de publication internationale: WO 2002/032462

(56) Documents cités:
- WO-A-00/27844
- WO-A-97/31691
- WO-A-99/59710
- WO-A1-88/08304
- WO-A1-95/01221
- WO-A1-98/36825
- WO-A1-01//03821
- WO-A1-96//00610
- WO-A1-96//29998
- WO-A2-01/15664
- WO-A2-99/47543
- FR-A1- 2 798 863
- US-A- 4 869 904
- US-A- 5 043 280
- SUBRANANIAM B ET AL: "PHARMACEUTICAL PROCESSING WITH SUPERCRITICAL CARBON DIOXIDE" JOURNAL OF PHARMACEUTICAL SCIENCES, AMERICAN PHARMACEUTICAL ASSOCIATION. WASHINGTON, US, vol. 86, no. 8, 1 août 1997 (1997-08-01), pages 885-890, XP000693966 ISSN: 0022-3549
- VAN HEES T ET AL: "Inclusion of piroxicam into beta- cyclodextrin by means of supercritical carbon dioxide: thermal, spectroscopic and physicochemical studies." JOURNAL DE PHARMACIE DE BELGIQUE, (2000 JAN-FEB) 55 (1) 30-1., XP001019795
- JUNG J ET AL: "Particle design using supercritical fluids: Literature and patent survey" JOURNAL OF SUPERCRITICAL FLUIDS, PRA PRESS, US, vol. 20, no. 3, août 2001 (2001-08), pages 179-219, XP004247117 ISSN: 0896-8446
- VAN HEES T ET AL: "Application of supercritical carbon dioxide for the preparation of a piroxicam-beta- cyclodextrin inclusion compound." PHARMACEUTICAL RESEARCH, (1999 DEC) 16 (12) 1864-70., XP001020308
- KAMIHIRA M ET AL: "FORMATION OF INCLUSION COMPLEXES BETWEEN CYCLODEXTRINS AND AROMATIC COMPOUNDS UNDER PRESSURIZED CARBON DIOXIDE." J FERMENT BIOENG, (1990) 69 (6), 350-353., XP001020305
- YORK, PETER: 'Strategies for particle design using supercritical fluid technologies' PHARMACEUTICAL SCIENCE TECHNOLOGY TODAY vol. 2, no. 11, Novembre 1999, pages 430 - 440
- WINTERS, CONRAD S. ET AL: 'Solid state examination of a gliclazide:beta-cyclodextrin complex' EUROPEAN JOURNAL OF PHARMACEUTICAL SCIENCES vol. 5, Janvier 1997, pages 209 - 214
- SUBRAMANIAM, BALA ET AL: 'Pharmaceutical Processing with Supercritical Carbon Dioxide' JOURNAL OF PHARMACEUTICAL SCIENCES vol. 86, no. 8, Août 1997, pages 885 - 890
- 1990, UNIVERSITY OF BRADFORD, BRADFORD article YUSUFF, NAHIMOT T.: 'Extracts from Ph D thesis'
- PERRUT, M. & SUBRA, P.: 'Proceedings of the 5th meeting on supercritical fluids, "Materials and natural products processing" Tome 1', Mars 1998 article SZE TU L., ET AL: 'Applications of dense gases in pharmaceutical processing', pages 263 - 269

## Description

La présente invention concerne un procédé de fabrication de très fines particules constituées d'au moins un principe actif inséré dans une molécule « hôte » notamment de type cyclodextrine, ainsi qu'un dispositif permettant de mettre en oeuvre ce procédé.

On sait que l'industrie pharmaceutique, mais également l'industrie des cosmétiques et l'agrochimie, requièrent de nouvelles formulations afin d'améliorer l'efficacité de certaines molécules d'intérêt thérapeutique, dermatologique ou phytosanitaire. Ces industries cherchent également des moyens permettant d'augmenter la solubilité dans les milieux biologiques de principes actifs insolubles, ou très peu solubles, afin d'augmenter leur bio-disponibilité, de diminuer les doses administrées et donc réduire les effets secondaires. Elles cherchent également à éviter la perte d'activité biologique due aux problèmes d'instabilité dans les milieux aqueux ou pendant le stockage en présence de l'oxygène, de l'humidité de l'air ou de la lumière. En vue de résoudre ces problèmes on a proposé d'utiliser différents excipients, sans toutefois aboutir à des solutions satisfaisantes.

On a proposé diverses méthodes permettant de fabriquer des fines particules constituées d'un principe actif qui est inséré dans une matrice, afin de le protéger contre des dégradations diverses, physiques ou chimiques, ou de faciliter sa solubilisation dans des solutions aqueuses.

On fait appel, depuis quelques années, à de nouvelles molécules hôtes qui sont aptes à former un complexe moléculaire dans lequel elles enserrent une molécule d'un principe actif. On connaît ainsi des molécules hôtes de type cyclodextrine qui connaissent actuellement un développement prometteur dans l'industrie pharmaceutique. Ces molécules, d'origine naturelle, issues de la dégradation enzymatique de l'amidon, sont produites industriellement et présentent l'avantage d'être biodégradables. Les cyclodextrines sont des molécules hôtes naturelles dont la forme cyclique leur permet de « capter » une grande variété de substances solides, liquides ou gazeuses conduisant à la formation de « supermolécules ».

Un tel « captage » des principes actifs est de façon générale mis à profit pour modifier les propriétés physico-chimiques des molécules captées et notamment leur solubilité, leur stabilité et leur réactivité. De plus, de nombreux groupements chimiques (méthyl-, hydroxypropyl-, carboxyméthyl-, acétyl- ) peuvent être greffés sur les cyclodextrines naturelles par réaction avec les groupements hydroxyles, modifiant les interactions avec les substances piégées. Le hasard de la position et du type de substitution rend amorphes ces cyclodextrines dites « modifiées », ce qui contribue à augmenter fortement leur solubilité dans l'eau et dans les solvants organiques. On peut ainsi citer la méthyl-β-cyclodextrine qui est très soluble dans l'eau (3000g/l), tout en n'étant pas hygroscopique ; elle ne change pas la tension de surface de l'eau où elle est dissoute et elle est également soluble dans le méthanol et l'éthanol. De plus, il est admis que cette molécule peut être utilisée en administration orale, parentérale, nasale, transdermale, vaginale ou rectale.

La présente invention concerne un nouveau procédé permettant de réaliser l'insertion au niveau moléculaire d'un principe actif, d'intérêt notamment pharmaceutique, cosmétologique, diététique ou phytosanitaire, dans une molécule hôte notamment de type cyclodextrine ou cyclodextrine modifiée et de réaliser de fines particules de ce complexe moléculaire, en utilisant un procédé mettant en oeuvre un fluide à pression supercritique.

On rappellera tout d'abord que les corps sont généralement connus sous trois états, à savoir solide, liquide ou gazeux et que l'on passe de l'un à l'autre en faisant varier la température et/ou la pression. Or il existe un point au-delà duquel on peut passer de l'état liquide à l'état de gaz ou de vapeur sans passer par une ébullition ou, à l'inverse, par une condensation, mais de façon continue. Ce point est appelé le point critique.

On sait également qu'un fluide en état supercritique, est un fluide qui est dans un état caractérisé soit par une pression et une température respectivement supérieures à la pression et à la température critiques dans le cas d'un corps pur, soit par un point représentatif (pression, température) situé au-delà de l'enveloppe des points critiques représentés sur un diagramme (pression, température) dans le cas d'un mélange. Un tel fluide présente, pour de nombreuses substances, un pouvoir solvant élevé sans commune mesure avec celui observé dans ce même fluide à l'état de gaz comprimé.

Il en est de même des liquides dits «subcritiques», c'est-à-dire des liquides qui se trouvent dans un état caractérisé soit par une pression supérieure à la pression critique et par une température inférieure à la température critique dans le cas d'un corps pur, soit par une pression supérieure aux pressions critiques et une température inférieure aux températures critiques des composants dans le cas d'un mélange. (cf. Michel PERRUT Les techniques de l'Ingénieur «Extraction par fluide supercritique, J 2 770 - 1 à 12, 1999»). On désignera dans la suite par fluide à pression supercritique un fluide porté à une pression supérieure à sa pression critique, qu'il soit en état supercritique ou subcritique comme défini ci-dessus.

Les variations importantes et modulables du pouvoir solvant des fluides à pression supercritique sont d'ailleurs utilisées dans de nombreux procédés d'extraction (solide/fluide), de fractionnement (liquide/fluide), de chromatographie analytique ou préparative, de traitement des matériaux (céramiques, polymères), de génération des particules, ou encore comme milieu de mise en oeuvre de réactions chimiques ou biochimiques. Il est à noter que les propriétés physico-chimiques du dioxyde de carbone ainsi que ses paramètres critiques (pression critique : 7,4 MPa et température critique : 31°C) en font un solvant préféré dans de nombreuses applications, d'autant qu'il ne présente pas de toxicité et est disponible à très bas prix en très grandes quantités. D'autres fluides peuvent également être utilisés dans des conditions voisines, comme le protoxyde d'azote, les hydrocarbures légers ayant deux à quatre atomes de carbone, et certains hydrocarbures halogénés.

Par de nombreux brevets et publications scientifiques, on sait que les fluides à pression supercritique, et particulièrement le dioxyde de carbone supercritique, sont largement utilisés pour réaliser des poudres très fines de tailles «microniques» ou «submicroniques» susceptibles de se dissoudre très rapidement ou qui sont utilisables par ingestion par les voies respiratoires. Les fluides à pression supercritique sont également étudiés en vue d'obtenir des particules complexes très fines formées de mélanges de différentes morphologies du principe actif et d'un excipient.

La plupart des systèmes décrits dans les brevets et publications s'appliquent à une encapsulation de type matricielle, qui consiste à intégrer une substance active dans un support notamment de type alginate, dérivés cellulosiques, cires, triglycérides, polysaccharides, ou polymères acryliques. On distinguera les microsphères, qui sont constituées d'un principe actif dispersé au sein d'un excipient des microcapsules, qui sont composées d'un coeur de substance active entouré par une enveloppe continue.

La technique dite « RESS » (Debenedetti P., Journal of Controlled Release, 24, 1993, p.27-44 - Debenedetti P., Journal of Supercritical Fluids, 7, 1994, p. 9-29) repose sur la mise en solution du principe actif et de l'excipient dans le fluide à pression supercritique. L'atomisation de cette solution supercritique permet la formation de microsphères. Cependant cette technique est limitée par la faible solubilité de la plupart des polymères et des substances actives dans les fluides supercritiques.

Les procédés dits anti-solvants, connus sous les désignations «SAS, SEDS, PCA, ou ASES» permettent la formation de micro-particules composites. Ils décrivent la mise en contact de solutions organiques de principes actifs et d'excipient avec un fluide supercritique. Différents brevets décrivent des divers moyens de mettre en contact les différents fluides : Introduction du fluide supercritique dans la solution organique (brevet US-A-5.360.478), pulvérisation séparée de la solution organique et du fluide supercritique (brevets DE-A-3.744.329, US-A-5.043.280), utilisation de buses coaxiales à deux ou trois entrées (brevets WO 95/01221, WO 96/00610).

Le procédé dit «PGSS» permet quant à lui l'encapsulation de principes actifs par pulvérisation à basse pression d'un mélange principe actif excipient saturé par un fluide supercritique (brevets EP-A-0744992, WO 95/21688).

Les fluides supercritiques permettent aussi de réaliser une encapsulation de type membranaire par inclusion de principes actifs dans des liposomes (brevet US-A- 5.700.482).

On connaît par un document Subrananiam B et al: "pharmaceutical processing with supercritical carbon dioxide" Journal of pharmaceutical sciences, american pharmaceutical association. Washington, US, qu'en remplaçant les solvants traditionnels par du dioxyde de carbone à l'état supercritique, et ceci afin de préserver l'environnement il est possible de produire de très fines particules contrôlées en dimension et en pureté.

On connaît également par les publications de Van Hees T et al: "Inclusion of piroxicam into beta-cyclodextrin by means of surpercritical carbon dioxide: thermal, spectroscopic and physicochemical studies." (Journal de pharmacie de Belgique) et de Jung J et al: "Particle design using supecritical fluids: Literature and patent survey" Journal of supercritical fluids, Pra Press, US, vol. 20, no. 3, août 2001 » un procédé de formation d'un complexe par insertion d'une molécule d'un principe actif (constitué de piroxicame) dans la cavité d'une molécule hôte (dans le cas présent de la cyclodextrine). Suivant ces deux publications, le principe actif est dissous dans un solvant constitué d'un fluide à pression supercritique, formé de dioxyde de carbone, et c'est cette solution qui est mise en contact avec la cyclodextrine au sein de laquelle elle diffuse, ce qui permet la formation d'un complexe constitué ici de piroxicam-cyclodextrine.

Dans la demande PCT WO 97/31691 on fait appel à un concept largement utilisé dans l'état antérieur de la technique pour assurer la préparation de particules très fines, en faisant appel à un anti-solvant supercritique ainsi que mentionné précédemment dans la description.

Dans la demande PCT WO 00/27844 on met en oeuvre une application particulière du même concept, afin de réaliser la préparation de particules très fines en utilisant un anti-solvant supercritique.

On connaît enfin par la publication Van Hees T et al: "Application of supercritical carbon dioxide for the préparation of a piroxicam-beta-cyclodextrin inclusion compound" (Pharmaceutical research, 1999 DEC), la formulation d'un complexe par insertion d'une molécule de principe actif dans la cavité d'une molécule hôte. Suivant ce procédé, le principe actif est dissous au sein d'un fluide à pression supercritique, et cette solution est mise en contact avec la molécule hôte, à savoir la cyclodextrine, au sein de laquelle elle diffuse, ce qui permet la formation d'un complexe piroxicam-cyclodextrine.

La présente invention a pour but de proposer un procédé de préparation de fines particules constituées d'au moins un principe actif, insoluble ou très peu soluble dans les solutions aqueuses, et qui est dispersé sous forme moléculaire dans des molécules hôtes de type cyclodextrine, utilisant un fluide à pression supercritique. Cette méthode permet d'éviter, ou de réduire à des quantités acceptables sur le plan toxicologique, les résidus de solvant organiques présents dans les particules obtenues.

Plus particulièrement, cette méthode permet de préparer des fines particules de principe actif, insolubles ou très peu solubles dans les solutions aqueuses, et qui sont insérées dans une molécule hôte de type cyclodextrine. Dans le cas de la formulation de produits pharmaceutiques, ces particules présentent une bio-disponibilité accrue du principe actif et ceci quel que soit le mode d'administration.

La présente invention a ainsi pour objet un procédé de fabrication de très fines particules contenant au moins un principe actif, ces particules étant formées d'un ensemble de complexes moléculaires chacun constitué d'une molécule de principe actif insérée dans une molécule hôte, caractérisé en ce qu'il comporte les étapes consistant à :
- mettre en solution le principe actif dans un premier solvant liquide et un produit formé des molécules hôtes dans un second solvant liquide,
- mettre en contact les solutions liquides ainsi formées avec un fluide à pression supercritique, de façon à diminuer le pouvoir solvant des solvants liquides et faire précipiter, par effet anti-solvant, les molécules hôtes qui y sont dissoutes,
- extraire les solvants résiduels au moyen d'un fluide à pression supercritique et évacuer le mélange fluide/solvants,
- récupérer les particules ainsi générées sous forme de poudre sèche.

Dans un mode de mise en oeuvre du procédé suivant l'invention dans lequel le principe actif est soluble dans le fluide à pression supercritique, ce dernier pourra être utilisé en tant que solvant. Préférentiellement, on assurera la saturation du fluide à pression supercritique en le faisant percoler à travers un lit de particules d'au moins un principe actif.

La présente invention a ainsi pour objet un procédé de fabrication de très fines particules contenant au moins un principe actif, ces particules étant formées d'un ensemble de complexes moléculaires chacun constitué d'une molécule de principe actif insérée dans une molécule hôte, caractérisé en ce qu'il comporte les étapes consistant à :
- mettre en solution le principe actif dans un premier solvant constitué d'un fluide à pression supercritique et un produit formé de molécules hôtes dans un second solvant liquide,
- mettre en contact les solutions ainsi formées de façon à diminuer le pouvoir solvant du solvant liquide et faire précipiter, par effet anti-solvant, les molécules hôtes qui y sont dissoutes,
- extraire les solvants résiduels au moyen d'un fluide à pression supercritique et évacuer le mélange fluide/solvants,
- récupérer les particules ainsi générées sous forme de poudre sèche.

Dans un mode de mise en oeuvre de l'invention les premier et second solvants seront identiques. La mise en solution du principe actif et du produit formé par les molécules hôtes et la mise en contact des solutions pourront également être effectuées au cours de la même étape.

Suivant l'invention la molécule hôte pourra être constituée d'au moins une cyclodextrine du type α-cyclodextrine, ou β-cyclodextrine ou γ-cyclodextrine. Elle pourra également être constituée d'au moins une cyclodextrine modifiée par greffage d'un groupement chimique, notamment du type méthyl-α-cyclodextrine, hydroxypropyl-α-cyclodextrine, méthyl-β-cyclodextrine, hydroxypropyl-β-cyclodextrine, carboxyméthyl-β-cyclodextrine, ou acétyl-β-cyclodextrine.

La présente invention a également pour objet un dispositif de fabrication de très fines particules comprenant au moins un principe actif inséré dans une molécule hôte caractérisé en ce qu'il comprend une chambre d'atomisation dont la partie supérieure est pourvue de moyens de pulvérisation qui sont alimentés de première part avec une solution liquide d'au moins un principe actif, de seconde part avec une solution liquide d'une matrice de type cyclodextrine, et de troisième part avec un fluide à pression supercritique, la partie inférieure de la chambre d'atomisation est pourvue de moyens de récupération des micro-particules formées et d'une sortie du fluide à pression supercritique qui est reliée à des moyens de séparation, notamment de type cyclonique, et des éléments de soutirage permettant un recyclage du fluide vers un réservoir de stockage.

Les moyens de pulvérisation pourront être constitués d'une buse permettant l'introduction simultanée de la solution d'au moins un principe actif, et de la solution liquide de matrice de cyclodextrine. Dans un mode de mise en oeuvre de l'invention la buse permettra en outre l'introduction simultanée du fluide à pression supercritique. Cette buse de pulvérisation pourra comporter un volume collecteur interne dans lequel convergent des canaux amont en communication avec les fluides que l'on souhaite pulvériser dans la chambre d'atomisation et un canal de sortie en communication avec cette chambre d'atomisation.

Dans la configuration habituelle, le procédé permet d'introduire séparément une solution d'un principe actif ou d'un mélange de principes actifs, une solution de molécules hôtes de type cyclodextrine, et un fluide à pression supercritique, la formation du complexe du ou des principes actifs et de la matrice ayant lieu dans un récipient sous pression, durant la phase de précipitation.

Selon une variante, le principe actif, ou le mélange de principes actifs, et le produit formé par les molécules hôtes, notamment de type cyclodextrine, sont dissous dans un même solvant liquide, et ces solutions peuvent être introduites en mélange dans le récipient sous pression balayé par le fluide à pression supercritique ; dans ce cas, le complexe principe(s) actif(s)-molécule hôte peut se former avant la mise en contact avec le fluide à pression supercritique ou durant la phase de précipitation.

Préférentiellement, les particules insérées auront un diamètre compris entre 0,01 µm et 30 µm et seront notamment constituées d'un principe actif d'intérêt alimentaire, pharmaceutique, cosmétique, agrochimique ou vétérinaire. Par ailleurs, et bien que l'on puisse utiliser un autre gaz, le fluide à pression supercritique sera favorablement constitué de dioxyde de carbone, éventuellement additionné d'un solvant organique volatil de type hydrocarbure léger, alcool, ester, cétone, ou halocarbone. Quel que soit leur diamètre on désignera ces particules dans le présent texte par fines particules.

On notera que le fluide à pression supercritique charge de solvants organiques pourra être recyclé selon les procédés classiquement utilisés en extraction-fractionnement supercritique, en particulier en utilisant des dispositifs du type de ceux décrits dans le brevet français FR-A-2 584 618.

D'autre part, la mise en contact du complexe principe actif-molécule hôte et d'un fluide à pression supercritique permet de réaliser simultanément la précipitation et le séchage des particules dans des conditions douces, ce qui en fait une technique de choix pour l'encapsulation de produits fragiles, comme par exemple les protéines.

Sur un plan pratique, la mise en contact du fluide supercritique avec la ou les solution(s) de principe(s) actif(s) et de molécule hôte, est soit effectuée par introduction du fluide à pression supercritique dans un autoclave contenant déjà la solution, soit par pulvérisation de(s) solution(s) à travers une ou plusieurs buses dans un autoclave balayé par un fluide à pression supercritique. Les buses utilisées peuvent avoir différentes configurations : entrées séparées de la solution ou des solutions et du fluide supercritique, ou entrée unique permettant la mise en contact des deux fluides juste avant l'orifice de la buse de façon à ce que la vitesse du fluide supercritique permette de pulvériser la solution liquide en très fines gouttelettes.

On décrira ci-après, diverses formes d'exécution de la présente invention, en référence au dessin annexé sur lequel :
La figure 1 est un schéma de principe d'une installation de production de fines particules d'au moins un principe actif encapsulé dans une molécule hôte de type cyclodextrine, suivant l'invention.
La figure 2 est une vue en coupe axiale d'un exemple de mise en oeuvre d'une buse de pulvérisation utilisée dans le dispositif suivant l'invention.

Ce dispositif est essentiellement constitué d'une chambre d'atomisation 1 dont la partie supérieure est pourvue d'une buse de pulvérisation 3 qui est alimentée en gaz liquéfié par une canalisation 5 reliée à un réservoir de stockage 7. Une pompe 9 et un échangeur 11 permettent de porter le gaz liquéfié à l'état supercritique. La solution du principe actif, ou du mélange de principes actifs, dans un solvant organique ou aqueux est contenue dans un récipient 13 et est amenée à la buse de pulvérisation 3 par une conduite 17 sous l'action d'une pompe 15. De même, la solution de molécule hôte, notamment de type cyclodextrine ou cyclodextrine modifiée, dans un solvant organique ou aqueux est contenue dans un récipient 19 et est amenée à la buse de pulvérisation 3 par une conduite 23 sous l'action d'une pompe 21. La partie inférieure de la chambre d'atomisation 1 est pourvue d'une sortie du fluide à pression supercritique 25 qui est reliée à des séparateurs cycloniques 31 par l'intermédiaire d'une vanne de régulation 27 et d'un filtre 29. La sortie des séparateurs 31 est reliée à des éléments de soutirage 33. Le dernier séparateur 31 est relié au réservoir de stockage 7 par une conduite 37 traversant un condenseur 35.

Suivant l'invention on injecte simultanément dans la chambre d'atomisation 1 les solutions de principe actif, ou du mélange de principes actifs contenus dans le récipient 13, la solution de la molécule hôte, contenue dans le récipient 19, et le gaz contenu dans le réservoir 7 qui est amené à l'état supercritique par la pompe 9 et l'échangeur 11.

Au cours de cette opération, le flux de fluide à pression supercritique entraîne le solvant dans lequel est dissous le principe actif et la molécule hôte sous forme de complexe ou non, ce qui a pour effet d'augmenter leur concentration au-delà de la saturation, provoquant ainsi la précipitation des produits sous forme complexée. Les fines particules obtenues sont séparées du fluide supercritique, contenant les solvants organiques ou aqueux, par passage au travers d'un élément filtrant 14, disposé au fond de la chambre d'atomisation 1, et qui est constitué, par exemple, d'un disque en métal fritté ou d'un textile tissé ou non-tissé.

En sortie de la chambre d'atomisation 1, le fluide chargé de solvant est partiellement détendu à la pression de recyclage à travers la vanne de régulation 27 et réchauffé dans les séparateurs cycloniques 31. après filtration à travers le filtre 29.

Le solvant collecté est soutiré en phase liquide à pression atmosphérique par les éléments de soutirage 33. Le fluide, débarrassé de la majeure partie du solvant est recyclé par liquéfaction dans le condenseur 35 vers le réservoir de fluide liquide 7. L'appoint de fluide à l'état liquide ou gazeux est réalisé par une entrée 8.

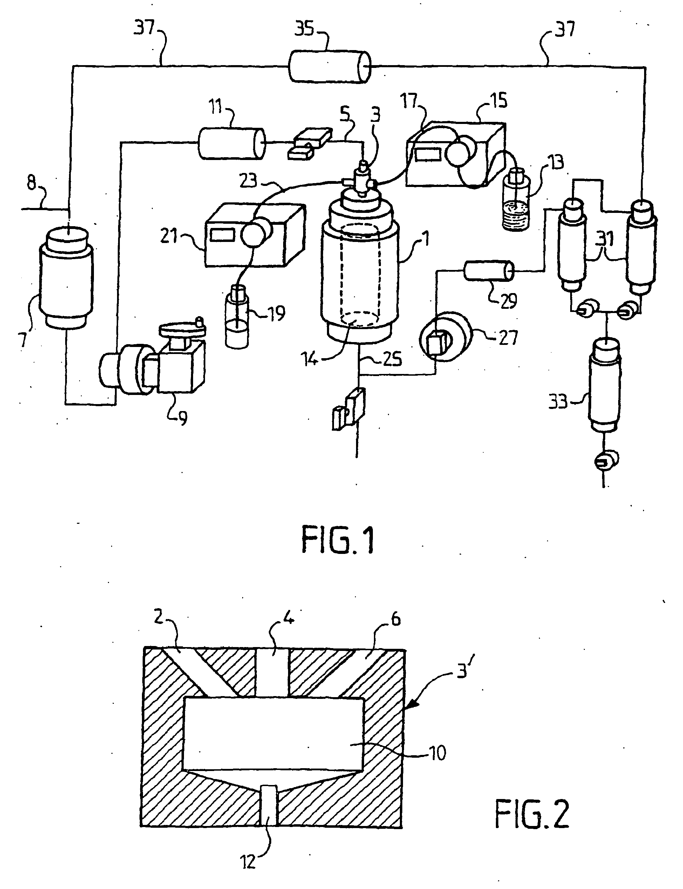

Selon une variante intéressante de l'invention particulièrement favorable à l'obtention de particules de très faible diamètre, on introduit les solutions de principe actif et de la molécule hôte ainsi que le fluide à pression supercritique par une buse 3' unique notamment du type de celle représentée sur la figure 2 et qui sera décrite ci-après.

Lorsque la quantité de fines particules fixées sur l'élément filtrant 14 est suffisante, on interrompt le pompage des solutions de principe actif et de molécule hôte. On peut ensuite éliminer les faibles quantités de solvant présent dans les fines particules en faisant percoler à travers le lit de ces particules déposées sur l'élément filtrant 14, un courant de dioxyde de carbone à l'état supercritique. Après élimination totale de ce solvant, la chambre d'atomisation 1 est dépressurisée et les fines particules récupérées sur l'élément filtrant 14.

Dans une variante de mise en oeuvre de l'invention on dissout dans le même solvant le principe actif, ou le mélange de principes actifs, et le produit constitué de molécules hôtes. Dans ce mode de mise en oeuvre une seule pompe 15 ou 21 est alors nécessaire pour injecter la solution dans la chambre d'atomisation 1 au moyen de la buse de pulvérisation 3.

La buse de pulvérisation 3' représentée sur la figure 2 est constituée d'une pastille métallique cylindrique dont la face principale amont est creusée de trois canaux 2, 4, 6, qui convergent dans une chambre collectrice 10 et qui assurent l'alimentation de celle-ci en solution de principe actif, en solution de la molécule hôte ainsi qu'en fluide à pression supercritique. Cette dernière est elle-même en communication avec la sortie aval de la buse 3' par une canalisation axiale 12.

Afin d'illustrer la présente invention, on citera les exemples de réalisation suivants mis en oeuvre sur une installation de taille pilote ayant une pression de service de 30 MPa et une gamme de température allant de 0°C à 150°C, construite selon le schéma présenté sur la figure 1.

Le dioxyde de carbone a été utilisé comme fluide à pression supercritique. La pompe à membrane 9 autorisait un débit de l'ordre de 6 kg/h à 20 kg/h de dioxyde carbone sous une pression de 30 MPa, les pompes d'alimentation des solutions 15 et 21 autorisaient un débit de 0,05 kg/h à 0,75 kg/h de liquide à 30 Mpa, le réservoir de fluide 7 ayant un volume total de 2 litres, la chambre d'atomisation 1 étant constituée d'un récipient tubulaire d'axe vertical, de diamètre 0,1 m et d'un volume total de 4 litres, doté sur sa section, au fond du récipient d'un élément filtrant 14 constitué d'une membrane en microfibres de verre non tissées d'une porosité de 0,7 µm supportée par un disque de métal fritté d'une porosité de 50 µm.

### Exemple 1 :

Au moyen de l'installation ainsi décrite, on a généré une poudre de particules très fines de complexe formé d'un stéroïde, la prédnisolone, et de méthyl-β-cyclodextrine, par pulvérisation d'une solution contenant 0,32 % en masse de prédnisolone et 2,5% en masse de méthyl-β-cyclodextrine, soit un rapport molaire de 1:2, dans l'éthanol absolu avec un débit de 0,5 kg/h dans un courant de 15 kg/h de dioxyde de carbone à 15 MPa et 40°C. Le principe actif et la matrice étant mis en solution dans l'éthanol concomitamment, on n'a utilisé qu'une seule pompe 15 d'introduction dans la chambre d'atomisation 1. Cette solution liquide et le fluide à pression supercritique ont été introduits dans la chambre 1 par une buse unique 3', les deux fluides se mélangeant dans la cavité 10 d'un volume voisin de 0,5 ml ménagée dans le corps de la buse 3', mise en communication avec la chambre d'atomisation 1 par le conduit 12 pourvu d'un orifice d'un diamètre de 0,06 mm.

Après une heure de pulvérisation, on a stoppé l'introduction de solution de principe actif et on a fait percoler un courant de dioxyde de carbone à 15 MPa et à 40°C dans le lit de particules fixées sur l'élément filtrant. Le dioxyde de carbone a été envoyé par la suite vers les séparateurs où l'on a récupéré le solvant organique. Après dépressurisation de la chambre d'atomisation 1, on a récupéré les micro-particules fixées sur l'élément filtrant 14.

Les caractéristiques des fines particules récupérées sont les suivantes :
- répartition granulométrique : 90% des particules ont un diamètre compris entre 0, 8 µm et 3,5 µm et un diamètre moyen de 1,4 µm.
- composition massique : 11% de prédnisolone et 85% de méthyl-β-cyclodextrine

La teneur des fines particules en solvant organique, déterminée par chromatographie en phase gazeuse de la phase aqueuse obtenue par agitation prolongée sous ultrasons de la poudre, est inférieure à 100 ppm ce qui autorise l'utilisation de ces particules sans traitement supplémentaire.

### Exemple 2

Au moyen de l'installation précédemment décrite, on a généré une poudre de particules très fines de complexe formé d'un anti-inflammatoire, l'ibuprofène, et de méthyl-β-cyclodextrine, par pulvérisation d'une solution contenant 0,22% en masse d'ibuprofène et 3,0% en masse de méthyl-β-cyclodextrine, soit un rapport molaire de 1:2, dans l'acétone avec un débit de 0,5 kg/h dans un courant de 15, kg/h de dioxyde de carbone à 15 MPa et 40°C. Après une heure de pulvérisation, on a stoppé l'introduction de solution de principe actif et on fait percoler un courant de dioxyde de carbone à 15 MPa et à 40°C dans le lit de particules fixées sur l'élément filtrant. Le dioxyde de carbone a été envoyé par la suite vers les séparateurs où l'on a récupéré le solvant organique. Après dépressurisation de la chambre d'atomisation, on a récupéré les fines particules fixées sur l'élément filtrant 14.

Les caractéristiques des fines particules récupérées sont les suivantes :
- répartition granulométrique : 90% des fines particules ont un diamètre compris entre 0,7 µm et 2.9 µm et un diamètre moyen de 1,1 µm.
- composition massique : 8% d'ibuprofène et 92% de méthyl-β-cyclodextrine.

La teneur des fines particules en solvant organique, déterminée par chromatographie en phase gazeuse de la phase aqueuse obtenue par agitation prolongée sous ultrasons de la poudre, est inférieure à 100 ppm ce qui autorise l'utilisation de ces fines particules sans traitement supplémentaire.

On a constaté que, bien que les molécules hôtes qui se révèlent les plus intéressantes sont les cyclodextrines et les cyclodextrines modifiées, on peut faire appel suivant l'invention à d'autres types de molécules hôtes telles que notamment les éthers-couronne.

## Revendications

1. Procédé de fabrication de très fines particules contenant au moins un principe actif, ces particules étant formées d'un ensemble de complexes moléculaires chacun constitué d'une molécule de principe actif insérée dans une molécule hôte, **caractérisé en ce qu'**il comporte les étapes consistant à :
- mettre en solution le principe actif dans un premier solvant liquide et un produit formé des molécules hôtes dans un second solvant liquide,
- mettre en contact les solutions liquides ainsi formées avec un fluide à pression supercritique, de façon à diminuer le pouvoir solvant des solvants liquides et faire précipiter, par effet anti-solvant, les molécules hôtes qui y sont dissoutes,
- extraire les solvants résiduels au moyen d'un fluide à pression supercritique et évacuer le mélange fluide/solvants,
- récupérer les particules ainsi générées sous forme de poudre sèche.

2. Procédé de fabrication de très fines particules contenant au moins un principe actif, ces particules étant formées d'un ensemble de complexes moléculaires chacun constitué d'une molécule de principe actif insérée dans une molécule hôte, **caractérisé en ce qu'**il comporte les étapes consistant à :
- mettre en solution le principe actif dans un premier solvant constitué d'un fluide à pression supercritique et un produit formé de molécules hôtes dans un second solvant liquide,
- mettre en contact les solutions ainsi formées de façon à diminuer le pouvoir solvant du solvant liquide et faire précipiter, par effet anti-solvant, les molécules hôtes qui y sont dissoutes,
- extraire les solvants résiduels au moyen d'un fluide à pression supercritique et évacuer le mélange fluide/solvants,
- récupérer les particules ainsi générées sous forme de poudre sèche.

3. Procédé suivant la revendication 2 **caractérisé en ce que** l'on sature en principe actif le fluide à pression supercritique, avant sa mise en contact avec la solution liquide.

4. Procédé suivant la revendication 3 **caractérisé en ce que** l'on obtient la saturation en principe actif du fluide à pression supercritique en le faisant percoler à travers un lit de particules d'au moins un principe actif.

5. Procédé suivant la revendication 1 **caractérisé en ce que** les premier et second solvants sont identiques.

6. Procédé suivant la revendication 5 **caractérisé en ce que** la mise en solution du principe actif et du produit formé par les molécules hôtes et la mise en contact des solutions ainsi formées sont effectuées au cours de la même étape.

7. Procédé suivant l'une des revendications précédentes **caractérisé en ce que** le fluide à pression supercritique est du dioxyde de carbone.

8. Procédé suivant l'une des revendications précédentes **caractérisé en ce que** la molécule hôte est constituée de cyclodextrine du type α-cyclodextrine, ou β-cyclodextrine ou γ-cyclodextrine.

9. Procédé suivant l'une des revendications 1 à 7 **caractérisé en ce que** la molécule hôte est constituée d'au moins une cyclodextrine modifiée du type méthyl-α-cyclodextrine, hydroxypropyl-α-cyclodextrine, ou méthyl-β-cyclodextrine, hydroxypropyl-β-cyclodextrine, ou carboxyméthyl-β-cyclodextrine, ou acétyl-β-cyclodextrine.

10. Procédé suivant l'une des revendications 1 ou 2, **caractérisé en ce que** la molécule hôte est constituée d'au moins une cyclodextrine modifiée par greffage d'un groupement chimique.

11. Procédé suivant l'une des revendications précédentes **caractérisé en ce que** le principe actif est un composé d'intérêt pharmaceutique, cosmétologique, diététique ou phytosanitaire.

## Claims

1. Method for making very fine particles containing at least one active principle, these particles being formed by an assembly of molecular complexes each constituted by a molecule of active principle inserted in a host molecule, **characterized in that** it comprises the steps consisting in:
- dissolving the active principle in a first liquid solvent and a product formed by the host molecules in a second liquid solvent,
- placing in contact the liquid solutions thus formed with a fluid at supercritical pressure, so as to reduce the solvent power of the liquid solvents and cause the host molecules which are dissolved therein to precipitate by anti-solvent effect,
- extracting the residual solvents by means of a fluid at supercritical pressure and evacuating the fluid/solvent mixture,
- recovering the particles thus generated in the form of dry powder.

2. Method for making very fine particles containing at least one active principle, these particles being formed by an assembly of molecular complexes each constituted by a molecule of active principle inserted in a host molecule, **characterized in that** it comprises the steps consisting in:
- dissolving the active principle in a first solvent constituted by a fluid at supercritical pressure and a product formed by host molecules in a second liquid solvent,
- placing in contact the solutions thus formed so as to reduce the solvent power of the liquid solvent and cause the host molecules which are dissolved therein to precipitate by anti-solvent effect,
- extracting the residual solvents by means of a fluid at supercritical pressure and evacuating the fluid/solvent mixture,
- recovering the particles thus generated in the form of dry powder.

3. Method according to Claim 2, **characterized in that** the fluid at supercritical pressure is saturated with active principle before it is placed in contact with the liquid solution.

4. Method according to Claim 3, **characterized in that** saturation of the fluid at supercritical pressure with active principle is obtained by causing it to percolate through a bed of particles of at least one active principle.

5. Method according to Claim 1, **characterized in that** the first and second solvents are identical.

6. Method according to Claim 5, **characterized in that** the dissolution of the active principle and of the product formed by the host molecules and the placing in contact of the solutions thus formed are effected in the course of the same step.

7. Method according to one of the preceding Claims, **characterized in that** the fluid at supercritical pressure is carbon dioxide.

8. Method according to one of the preceding Claims, **characterized in that** the host molecule is constituted by cyclodextrin of α-cyclodextrin, or β-cyclodextrin or γ-cyclodextrin type.

9. Method according to one of Claims 1 to 7, **characterized in that** the host molecule is constituted by at least one modified cyclodextrin of the methyl-α-cyclodextrin, hydroxypropyl-α-cyclodextrin, or methyl-β-cyclodextrin, hydroxypropyl-β-cyclodextrin, or carboxymethyl-β-cyclodextrin, or acetyl-β-cyclodextrin type.

10. Method according to one of Claims 1 or 2, **characterized in that** the host molecule is constituted by at least one cyclodextrin modified by grafting a chemical group.

11. Method according to one of the preceding Claims, **characterized in that** the active principle is a compound of pharmaceutical, cosmetological, dietetic or plant-protective interest.

## Patentansprüche

1. Verfahren zur Herstellung von sehr feinen Teilchen, die mindestens einen Wirkstoff enthalten, wobei diese Teilchen aus einer Anordnung von Molekülkomplexen gebildet sind, von denen jeder aus einem Wirtsmolekül mit inseriertem Wirkstoff besteht, **dadurch gekennzeichnet, dass** es folgende Schritte umfasst:
- In-Lösung-Bringen des Wirkstoffes in einem ersten flüssigen Lösungsmittel und eines aus Wirtsmolekülen aufgebautes Produktes in einem zweiten flüssigen Lösungsmittel,
- In-Kontakt-Bringen der auf diese Weise erzeugten flüssigen Lösungen mit einem auf überkritischem Druck befindlichen Fluid, so dass das Lösungsvermögen der flüssigen Lösungsmittel verringert wird und durch den löslichkeitsverringernden Effekt die darin gelösten Wirtsmoleküle ausgefällt werden,
- Extrahieren der verbleibenden Lösungsmittel mittels eines auf überkritischem Druck befindlichen Fluids und Evakuieren das Fluid/LösungsmittelGemisches,
- Gewinnen der auf diese Weise erzeugten Teilchen in Form von trockenem Pulver.

2. Verfahren zur Herstellung von sehr feinen Teilchen, die mindestens einen Wirkstoff enthalten, wobei diese Teilchen aus einer Anordnung von Molekülkomplexen gebildet sind, von denen jeder aus einem Wirtsmolekül mit inseriertem Wirkstoff besteht, **dadurch gekennzeichnet, dass** es folgende Schritte umfasst:
- In-Lösung-Bringen des Wirkstoffes in einem ersten Lösungsmittel, das aus einem unter überkritischem Druck stehenden Fluid besteht, und eines aus Wirtsmolekülen aufgebautes Produktes in einem zweiten flüssigen Lösungsmittel,
- In-Kontakt-Bringen der auf diese Weise erzeugten Lösungen, so dass das Lösungsvermögen des flüssigen Lösungsmittels verringert wird und durch den löslichkeitsverringernden Effekt die darin gelösten Wirtsmoleküle ausgefällt werden,
- Extrahieren der verbleibenden Lösungsmittel mittels eines auf überkritischem Druck befindlichen Fluids und Evakuieren das Fluid/LösungsmittelGemisches,
- Gewinnen der auf diese Weise erzeugten Teilchen in Form von trockenem Pulver.

3. Verfahren nach Anspruch 2, **dadurch gekennzeichnet, dass** das unter überkritischem Druck stehende Fluid mit dem Wirkstoff gesättigt wird, bevor es mit der flüssigen Lösung in Kontakt gebracht wird.

4. Verfahren nach Anspruch 3, **dadurch gekennzeichnet, dass** die Sättigung des unter überkritischem Druck stehenden Fluids mit dem Wirkstoff **dadurch** erzielt wird, dass man es durch ein Bett aus Teilchen mindestens eines Wirkstoffes perkolieren lässt.

5. Verfahren nach Anspruch 1, **dadurch gekennzeichnet, dass** das erste und das zweite Lösungsmittel identisch sind.

6. Verfahren nach Anspruch 5, **dadurch gekennzeichnet, dass** das In-Lösung-Bringen des Wirkstoffs und des durch die Wirtsmoleküle gebildeten Produktes und das In-Kontakt-Bringen der auf diese Weise erzeugten Lösungen in demselben Schritt durchgeführt wird.

7. Verfahren nach einem der vorhergehenden Ansprüche, **dadurch gekennzeichnet, dass** das unter überkritischem Druck stehende Fluid Kohlendioxid ist.

8. Verfahren nach einem der vorhergehenden Ansprüche, **dadurch gekennzeichnet, dass** das Wirtsmolekül aus Cyclodextrin vom Typ α-Cyclodextrin oder β-Cyclodextrin oder γ-Cyclodextrin besteht.

9. Verfahren nach einem der Ansprüche 1 bis 7, **dadurch gekennzeichnet, dass das** Wirtsmolekül aus mindestens einem modifizierten Cyclodextrin vom Typ Methyl-α-Cyclodextrin, Hydroxypropyl-α-Cyclodextrin oder Methyl-β-Cyclodextrin, Hydroxypropyl-β-Cyclodextrin oder Carboxymethyl-β-Cyclodextrin oder Acetyl-β-Cyclodextrin besteht.

10. Verfahren nach einem der Ansprüche 1 oder 2, **dadurch gekennzeichnet, dass** das Wirtsmolekül aus mindestens einem Cyclodextrin besteht, das durch Einpflanzen einer chemischen Gruppe modifiziert wurde.

11. Verfahren nach einem der vorhergehenden Ansprüche, **dadurch gekennzeichnet, dass** der Wirkstoff eine Verbindung ist, die von pharmazeutischem, kosmetischem, diätetischem Interesse oder für den Pflanzenschutz von Interesse ist.
